# EUROPEAN PATENT APPLICATION

(11) **EP 4 588 423 A1**
(43) Date of publication of application: **23.07.2025**
(21) Application number: 25150597.0
(22) Date of filing: 08.01.2025
(51) Int. Cl.: A61B 1/04, A61B 1/06, A61B 1/303, A61B 1/31, A61B 1/32

(54) **DEVICE AND KIT FOR INSPECTING A CAVITY**

(30) Priority: 19.01.2024 IT 202400000978
(71) Applicant: THD S.p.A., 42015 Correggio, Reggio Emilia (IT)
(72) Inventor: BASTIA, FILIPPO, 41019 SOLIERA (IT)
(74) Representative: Paparo, Aldo

(57) **Abstract**

A device (1) for inspection of a cavity, comprising a dilator body (2) and a gripping element (4) with an elongated shape, applied or applicable to the end of the dilator body (2), arranged transversely with respect to the dilator body (2) and graspable by an operator. The device (1) comprises a support element (5), configured to define a reversible connection with an inspection group (1000), connected to the gripping element (4) and movable along the gripping element (4) towards and/or away from the dilator body (2). The inspection group (1000) comprises an acquisition device and a lighting device.

## Description

The present invention relates to a device for the inspection of a cavity and a kit for the inspection of a cavity comprising the device.

The present invention is advantageously applied in the sector of diagnosis of pathologies relating to human, natural or surgically obtained cavities or orifices, a diagnosis that is performed by means of visual inspection of the tissues delimiting the cavities and the orifices.

In particular, but not exclusively, the present invention is advantageously applied in the sector of anorectal and vaginal inspection. Consequently, the device that is the object of the present invention preferably, but not exclusively, has the alternative embodiments of anoscope, proctoscope, rectoscope and vaginal speculum.

Inspection devices comprising a dilator body and an introducer body are known in the prior art.

The dilator body is hollow and has a tubular extension between a distal opening, to visually and operatively access the mucous membranes delimiting the cavity, and a proximal opening, accessible to an operator at the moment of use.

In detail, the introducer body also has a tubular extension, between a distal end with a substantially ogival shape and a proximal end, graspable by the operator.

The introducer body is configured to be coupled to the dilator body by sliding of the introducer body inside the dilator body, until the ogival end of the introducer body protrudes from the distal end of the dilator body, obtaining outer walls of the device shaped to facilitate its insertion into the cavity of the patient.

During an inspection examination, after coupling between the dilator body and the introducer body, the operator inserts the inspection device into the cavity of the patient, and then extracts the introducer body from the dilator body, maintaining the dilator body inserted inside the cavity and allowing visual access and, therefore, inspection of the mucous membrane through the distal opening (with respect to the operator) of the dilator body.

The known dilator bodies can be coupled with detection instruments, such as cameras or illuminators, configured respectively for the detection of images and for lighting of the cavity being inspected.

It is currently known to obtain such coupling directly at the proximal opening (with respect to the operator) once the dilator body is correctly positioned in the cavity of the patient.

This procedure, in addition to being not very functional for the operator, is uncomfortable for the patient.

In order to overcome these drawbacks, it is known to provide the inspection device with a support coupled to an illuminator, arranged directly inside the volume defined by the dilator body.

In other words, the support is positioned between the dilator body and the introducer body, so that an operator, once the inspection device has been inserted into the cavity of the patient and the introducer body has been extracted, can proceed with inspection without the need to manually couple the dilator body with the external supports.

Disadvantageously, these solutions do not provide for the use of cameras, thus precluding the possibility of acquiring diagnostic images. Furthermore, disadvantageously, the known solutions are not very practical in the usual case in which the operator has to inspect the entire mucous membrane facing the dilator body and the distal opening of the dilator body.

The result is, disadvantageously, both an inspection procedure that is not very functional for the operator and a lengthy inspection examination for a patient.

In addition, the known solutions disadvantageously prevent the operator from having an overall view of the mucous membrane facing the distal opening of the dilator body and/or adjacent to the side walls of the dilator body. The known solutions therefore make the inspection examination difficult.

In addition, disadvantageously, the known solutions are not very sustainable, since the illuminator arranged inside the dilator body, as it is inserted together with it into the cavity of the patient, needs to be replaced, in particular discarded together with the inspection device, after every use. Therefore, the known devices are not only not very practical, but also costly and poorly sustainable.

The technical task of the present invention is thus to provide an inspection device and kit that are able to overcome the drawbacks that have emerged from the prior art.

An object of the present invention is to provide an inspection device and kit that are functional and practical to use for an operator.

Another object of the present invention is to provide an inspection device and kit at reduced cost.

Another object of the present invention is to provide a sustainable inspection device and kit.

The specified technical task and the specified objects are substantially achieved by an inspection device and kit comprising the technical features disclosed in one or more of the appended claims.

In particular, the device comprises a dilator body, having a main extension along a longitudinal axis between a proximal end and a distal end.

The proximal end and the distal end are connected by a side wall and define, together with the side wall, an inner volume.

In more detail, the proximal end identifies a proximal opening, and the distal end identifies a distal opening for access, from the inner volume, to the tissues delimiting the cavity.

The device comprises an introducer body, applicable in use in a removable manner to the dilator body, in particular the introducer body is slidably insertable into, and disengageable from, the inner volume through the proximal opening.

The device comprises a gripping element with an elongated shape, applied or applicable to the proximal end of the dilator body and arranged transversely with respect to the longitudinal axis.

The gripping element is graspable by an operator.

The device comprises a support element, arranged externally with respect to the inner volume defined by the dilator body, so as to support the inspection group externally with respect to said inner volume, and movable along said gripping element towards and/or away from the proximal end of the dilator body.

In detail, the inspection group comprises at least one acquisition device and at least one lighting device.

Advantageously, the device is versatile, since an operator, in order to perform an inspection examination, must connect the inspection group to the support element, without needing to connect external supports to the proximal opening of the inspection device.

Furthermore, thanks to the support element, the device allows the operator to have an overall view of the mucous membrane of the patient. Advantageously, the device is also versatile since the operator can position the inspection group with respect to the proximal opening by taking action on the support element.

Advantageously, the device is sustainable and economical, since at least the inspection group is reusable, as it is supported externally with respect to the inner volume defined by the dilator body.

Preferably, the device comprises a guide element, defined on the gripping element or applied in a removable manner to the gripping element, configured to guide a movement of the support element along a predefined path, extending along the gripping element itself. Advantageously, the guide element allows facilitated adjustment of the positioning of the inspection group with respect to the proximal opening of the dilator body.

Preferably, the guide element comprises a plurality of locking members defining a respective plurality of predefined positions in which to retain the support element along the gripping element.

Preferably, the locking members comprise protuberances defined on the guide element.

Advantageously, the locking members allow an immediate adjustment of the positioning of the inspection group with respect to the proximal opening of the dilator body.

Preferably, the support element is slidable along the gripping element between an insertion position, in which the support element is arranged in a distal position with respect to the proximal opening, an acquisition position, in which the support element is arranged in a proximal position with respect to the proximal opening, and an observation position, in which the support element is arranged between the insertion position and the acquisition position.

Advantageously, the insertion position allows the dilator body to be inserted in a facilitated manner inside the cavity of the patient, since the support element is arranged in a distanced position from it. Advantageously, the acquisition position allows images of the cavity of the patient to be acquired in a facilitated manner, since, thanks to the support element that supports the inspection group, the operator can observe the images acquired without worrying about the positioning of the inspection group, and in particular of the acquisition device.

Advantageously, the observation position allows the operator to observe with the naked eye the cavity of the patient in a facilitated manner, since, thanks to the support element that supports the inspection group, the operator can observe the cavity without worrying about the positioning of the inspection group, and in particular of the lighting device. Advantageously, the device allows the cavity of the patient to be inspected using different methods (naked eye, on-screen), allowing an in-depth inspection examination.

The present invention also provides an inspection kit of a cavity.

The kit comprises the device, the inspection group and a portable battery connected or connectible to the inspection group.

Therefore, the kit comprises the device, at least one acquisition device, at least one lighting device and a portable battery connected or connectible to the acquisition device and to the lighting device.

The dependent claims correspond to possible embodiments of the invention.

Further features and advantages of the present invention will become more apparent from the indicative and thus non-limiting description of an embodiment of an inspection device and an inspection kit comprising the device.

Such a description will be set out below with reference to the accompanying drawings, which are provided solely for illustrative and therefore non-limiting purposes, in which:
- figure 1 shows a schematic and prospective view of the inspection kit in accordance with the present invention;
- figure 2 shows a schematic view in longitudinal section of the kit of figure 1, in which the support element is in the insertion position;
- figure 3 shows a schematic view in longitudinal section of the kit of figure 2 in which the support element is in the observation position;
- figure 4 shows a schematic view in longitudinal section of the kit of figure 2 in which the support element is in the acquisition position;
- figure 5 shows a front prospective schematic and exploded view of the kit of figure 1;
- figure 6 shows an exploded rear view of figure 5.

With reference to the appended figures, the reference number 1 denotes in its entirety an inspection device that, in this description will be indicated, for simplicity, as "device 1".

The device 1 is preferably an anoscope, a proctoscope or a rectoscope, for the inspection of an anal or rectal cavity of a patient.

Alternatively, the device 1 is a vaginal speculum, for the inspection of the vaginal cavity of a patient.

However, devices suitable for the inspection of different cavities, natural or surgically obtained in the patient, are not excluded.

The terms "distal" and "proximal" will be used in this description. These terms are, unless otherwise specified, to be intended as with respect to the operator. Therefore, unless otherwise specified, the term "proximal" is to be intended as referring to the parts furthest away from the patient, whereas the term "distal" is to be intended as referring to the parts closest to the patient.

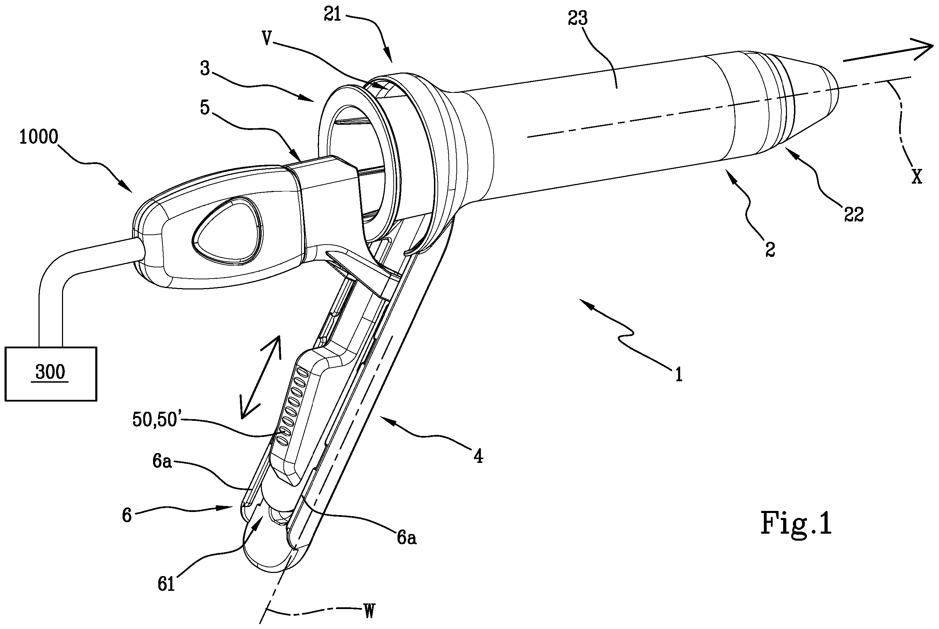

With reference to figures 1, 5 and 6, the device 1 according to the present invention comprises a dilator body 2 and an introducer body 3.

The dilator body 2 is shaped to be inserted into the cavity of the patient on whom it is intended to perform the inspection.

The dilator body 2 has an elongated development along a longitudinal axis "X", in particular between a proximal end 21 and a distal end 22, connected by a side wall 23.

In other words, the dilator body 2 has a substantially tubular shape.

In a non-exclusive embodiment, the side wall 23 extends in a diverging fashion from the distal end 22 to the proximal end 21.

Advantageously, the diverging extension of the side wall 23 facilitates dilation of the cavity to be inspected and, therefore, insertion of the dilator body 2 into the cavity itself.

The side wall 23 is preferably made from transparent material, more preferably from plastic material.

In detail, the side wall 23 delimits and identifies, together with the proximal end 21 and the distal end 22, an inner volume "V".

The proximal end 21 of the dilator body 2 identifies a respective proximal opening "PO", facing the inner volume "V" and accessible by the operator. In a non-exclusive embodiment, the proximal opening "PO" is coaxial with the longitudinal axis "X" along which the dilator body 2 extends.

Similarly, the distal end 22 identifies a respective distal opening "DO", facing the inner volume "V" and configured to allow access, from the inner volume "V", to the tissues and to the mucous membranes delimiting the cavity.

Preferably, the distal opening "DO" is coaxial with the longitudinal axis "X". Preferably, the proximal opening "PO" and the distal opening "DO" have a substantially circular shape.

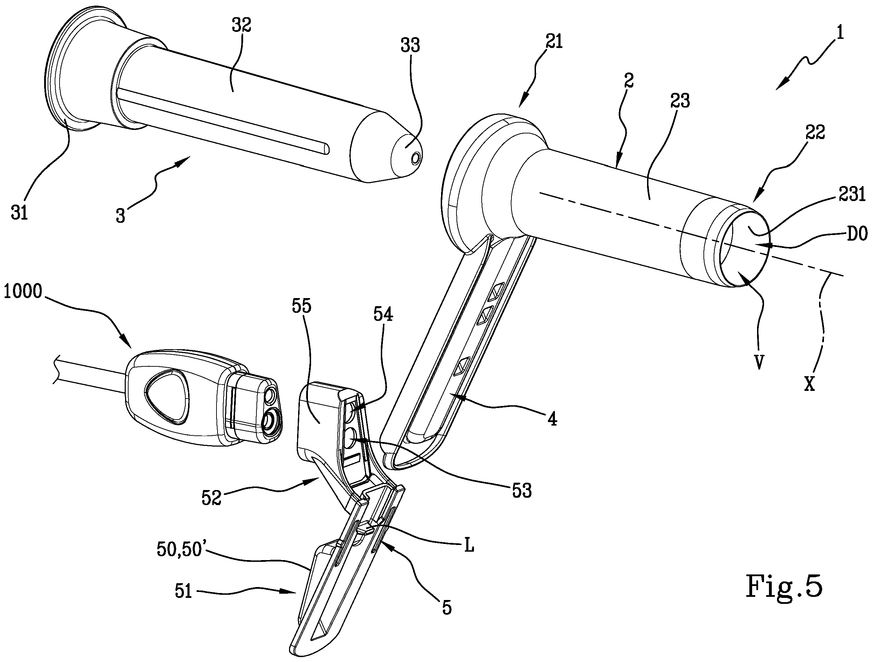

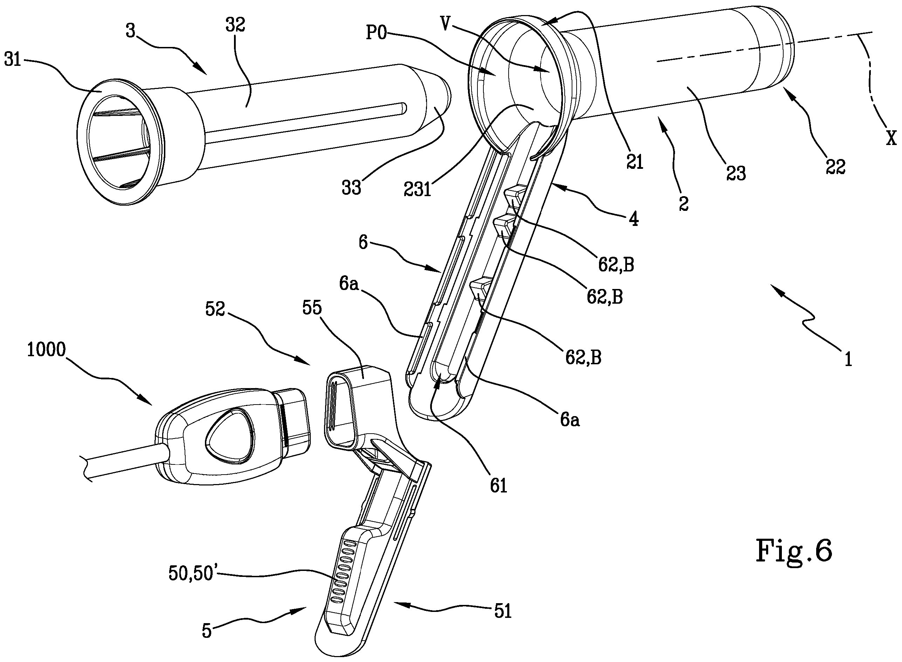

With reference to the introducer body 3, it comprises a gripping portion 31 and an insertion portion 32 (as shown in figures 5 and 6).

The gripping portion 31 is accessible to or graspable by the operator, in detail, the gripping portion 31 is shaped to allow the operator to grasp and manoeuvre the introducer body 3.

The insertion portion 32 is connected to the gripping portion 31 and has an elongated extension along a respective axis of extension.

In particular, the insertion portion 32 extends between an insertion end 33, with a substantially ogival shape and distal with respect to the gripping portion 31, and the gripping portion 31.

The insertion portion 32 is configured to be inserted into the inner volume "V" identified by the dilator body 2, to obtain an assembled configuration of the device 1, shown in figures 1-4, which can be easily inserted into the cavity to be explored.

In order to allow easy insertion of the dilator body 2 inside the cavity to be explored, the dilator body 2 and the introducer body 3 are thus assembled. In detail, the assembled configuration is obtained through insertion of the introducer body 3 into the inner volume "V" of the dilator body 2 through the proximal opening "PO", preferably along the longitudinal axis "X". Insertion of the introducer body 3 inside the inner volume "V" of the dilator body 2 occurs through sliding of the insertion portion 32 inside the dilator body 2, in particular along an inner surface 231 delimiting internally the side wall 23 of the dilator body 2. In other words, the inner surface 231 is a surface delimiting the side wall 23 and facing the inner volume "V".

For example, but not exclusively, the inner surface 231 can have one or more sliding guides (not shown in the appended figures) and the insertion portion 32 can be coupled with the aforesaid sliding guides. Therefore, the sliding guides allow sliding of the insertion portion 32 to be guided along the inner surface 231, advantageously guaranteeing a facilitated insertion of the introducer body 3 into the inner body "V".

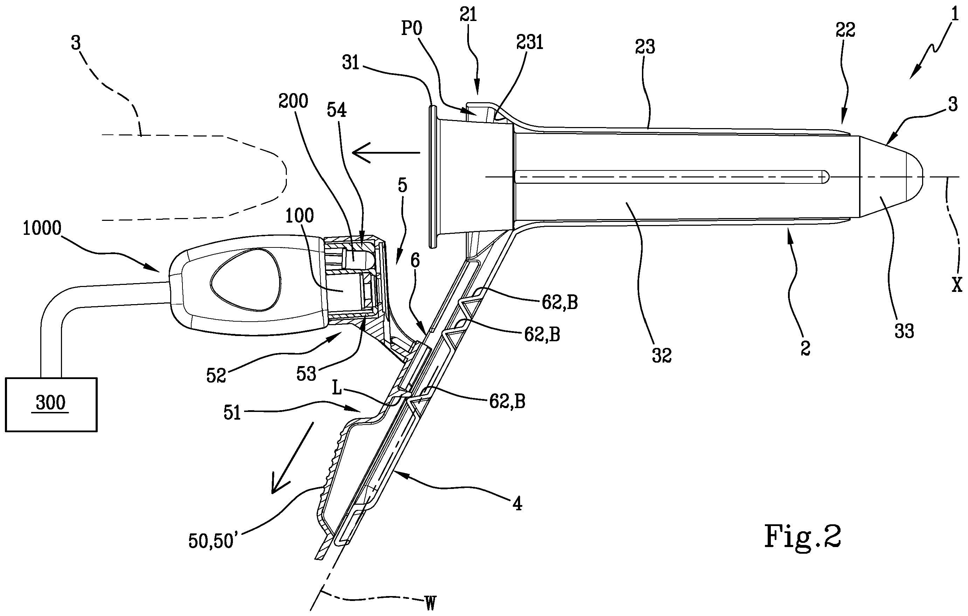

In particular, the insertion portion 32 is introduced into the inner volume "V" of the dilator body 2 up to an end-of-travel position, in which the insertion end 33 is protruding from the distal opening "DO" of the dilator body 2 as shown in figures 1 and 2.

The result is that the device 1, in the assembled configuration, has an elongated extension, diverging from the insertion end 33 of the introducer body 3 to the proximal end 21 of the dilator body 2.

In addition, the device 1 in the assembled configuration, outside the inner volume "V", has no edges or discontinuous elements that could make insertion and positioning of the device 1 in the cavity of the patient uncomfortable or painful.

In accordance with a non-exclusive embodiment, the introducer body 3 can comprise locking elements (not shown in the appended figures), configured to reversibly constrain the introducer body 3 to the dilator body 2 when the introducer body 3 is completely inserted inside the dilator body 2.

The aforesaid locking elements, of the type known in the art, therefore allow the introducer body 3 to be maintained constrained to the dilator body 2 during insertion of the device 1.

In other words, thanks to the locking elements, the dilator body 2, together with the introducer body 3 inserted inside the inner volume "V", can be advantageously inserted inside the cavity of a patient by the operator using a single hand, without running the risk of the introducer body 3, during insertion into the cavity, being moved with respect to the dilator body 2 in a different direction to the insertion one, as a result of the resistance forces exerted, for example, by the muscle structures surrounding the cavity of the patient.

When insertion into the cavity of the patient has been completed, the introducer body 3 is removed from the dilator body 2 as shown schematically in figure 2, following an extraction trajectory, opposite with respect to the insertion trajectory.

In other words, the insertion portion 32 of the introducer body 3 is moved parallel to the longitudinal axis "X", causing the extraction through sliding of the introducer body 3, in particular of the insertion portion 32, from the inner volume "V".

The device 1 comprises a gripping element 4 applied or applicable to the proximal end "PO" of the dilator body 2.

In a non-exclusive embodiment shown in the appended figures, the gripping element 4 defines, with the dilator body 2, a single monolithic body.

Alternatively, in another non-exclusive embodiment, the gripping element 4 is connected in a removable manner to the proximal end "PO" of the dilator body 2.

In particular, the gripping element 4 has an elongated shape, extending along a main extension axis "W" that is transverse with respect to the longitudinal axis "X".

In other words, the gripping element 4 is arranged transversely with respect to the dilator body 2.

The gripping element 4 is also graspable by an operator.

In other words, the gripping element 4 is a handle, inclined by an angle comprised between 1° and 90° with respect to the longitudinal axis "X".

In accordance with an advantageous aspect of the present invention, the device 1 comprises a support element 5, configured to define a reversible connection with an inspection group 1000, in particular provided with at least one acquisition device 100 and at least one lighting device 200.

The inspection group 1000 may, for example, be defined by a single body, provided with the acquisition 100 and lighting 200 devices.

The support element 5 is arranged externally with respect to the inner volume "V" and is movable along the gripping element 4 towards and away from the dilator body 2.

Preferably, in order to allow a facilitated movement of the support element 5 along the gripping element 4, the support element 5 comprises an activation zone 50, graspable by the operator.

For example, as shown in the appended figures, the activation zone 50 may be obtained in the form of a slider 50' configured to allow the operator to activate a sliding of the support element 5 along the gripping element 4 through an action of a finger on the slider 50'.

Preferably, in order to define the connection with the acquisition device 100, the support element 5 comprises an acquisition seat 53 (shown in figures 2-5), configured to receive the acquisition device 100 by insertion. Preferably, in order to define the connection with the lighting device 200, the support element 5 comprises a lighting seat 54 (shown in figures 2-5), configured to receive the acquisition device 100 by insertion.

In more detail, the support element 5 is arranged externally with respect to the inner volume "V" so as to support the inspection group 1000 externally with respect to said inner volume "V".

The result is that, thanks to the support element 5, the inspection group 1000, i.e. the acquisition device 100 and the lighting device 200, during the inspection examination, i.e. when the dilator body 2 is inserted inside the cavity of a patient, are positioned outside the cavity itself.

In other words, thanks to the support element 5, the acquisition device 100 and the lighting device 200 do not come into contact with the mucous membrane of the patient, or with any secretions produced by it. Therefore, advantageously, the device 1 is sustainable, since it does not require replacement and disposal of the inspection group 1000 after every use.

In detail, the support element 5 is connected to the gripping element 4 and is movable along the gripping element 4 towards and/or away from the dilator body 2.

The result is that the operator, once the dilator body 2 has been inserted into the cavity of the patient and the introducer body 3 has been removed from the inner volume "V", must connect the inspection group 1000 directly to the support element 5, without the need, difficult for the operator and uncomfortable as well as painful for the patient, to couple the proximal opening "PO" with external supports when the dilator body 2 is inserted into the cavity of the patient.

Furthermore, in order to inspect/film the cavity of the patient in greater depth, the operator can position the support device 5 (and therefore the acquisition device 100 and the lighting device 200) as they desire with respect to the dilator body 2, and in particular with respect to its proximal opening "PO", for example by acting on the slider 50'.

Therefore, advantageously, thanks to the support element 5, the device 1 is versatile to use.

In accordance with an aspect of the present invention, the device comprises a guide element 6, arranged at the gripping element 4.

In particular, the guide element 6 is configured to guide a movement of the support element 5 along a predefined path, extending along the gripping element 4.

In other words, thanks to the guide element 6, it is possible for an operator to move the support element 5 in a facilitated manner during performance of an inspection examination on a patient.

Preferably, the predefined path extends parallel to the main extension axis "W" of the gripping element 4.

In accordance with an aspect of the present invention, the guide element 6 is defined on the gripping element 4.

In other words, the guide element 6 and the gripping element 4 define a single monolithic body.

For example, the guide element 6 is obtained in the form of at least one rail 6a (as shown in figures 1 and 6) defined on the gripping element 5, in particular along its main extension axis "W".

Alternatively, the guide element 6 is applied or applicable in a removable manner to the gripping element 4, in other words, the guide element 6 and the gripping element 5 are connectible to or disconnectible from each other.

For example, the guide element 6 is obtained in the form of at least one rail 6a, applied in a removable manner to the gripping element 4, in particular along its main extension axis "W".

In accordance with an aspect of the present invention, the guide element 6 defines a respective channel 61 delimiting the predefined path.

For example, the guide element 6 is a recess obtained on the gripping element 5 along the main extension axis "W", or (in the non-exclusive embodiment shown in figures 1 and 6), the channel 61 is delimited by a pair of rails 6a, applied or applicable to the gripping element, facing and parallel to each other.

In more detail, the support element 5 comprises an interconnection portion 51, for connection with the gripping element 4, in particular with the guide element 6, and a connection portion 52, for connection with the inspection group 1000.

The interconnection portion 51, in particular, is inserted into the channel 61 and is sliding along the channel 61 itself and/or coupled with the rail 6a. For example, the interconnection portion 51 is obtained in the form of a pin, intended to be inserted into the channel 61 and to slide along the edges of the channel 61 itself, i.e. along the rails 6a.

Preferably, the interconnection portion 51 comprises the slider 50' accessible to the operator, for example in an opposite position to the channel 61.

The connection portion 52 is, on the other hand, arranged externally with respect to the channel 61, and is configured to support the acquisition device 100 and the lighting device 200 externally with respect to the channel 61.

For example, the connection portion 52 may comprise a bracket, intended to receive the inspection group 1000, and an upright connected to the interconnection portion 51 and to the connection portion 52. Alternatively, the connection portion 52 may comprise a first bracket, suitable to support the acquisition device 100, and a second bracket, suitable to support the lighting device 200.

In accordance with an embodiment shown in the appended figures, the acquisition seat 53 and the lighting seat 54 are defined on the connection portion 52, and in detail are pass-through with respect to the connection portion 52.

In accordance with a non-exclusive embodiment, the support element 5 comprises an adaptor 55, configured to define a reversible connection with the inspection group 1000, i.e. with the acquisition device 100 and with the lighting device 200.

Therefore, the adaptor 55 comprises the acquisition 53 and lighting 54 seats.

In accordance with a non-exclusive embodiment, the adaptor 55 is removable from the support element 5, for example, the support element 5 may comprise, in its connection portion 52, an insertion seat for the adaptor 55.

In accordance with a further variant, the adaptor 55 is connectible to and disconnectible from the interconnection portion 51 of the support element 5.

In accordance with a further variant, the connection portion 52 defines the adaptor 55.

In accordance with an advantageous aspect of the present invention, the guide element 6 comprises locking members "B" configured to define a plurality of predefined positions in which to retain the support element 5 along the gripping element 4.

In other words, the support element 5 is configured to move "in jerks" along the gripping element 4 at least between a first locking member "B" and a second locking member "B" directly successive to the first. Therefore, the locking members "B" are distributed along the gripping element 4, i.e. along the guide element 6.

In other words, the support element 5 is configured to move between the successive locking members "B" with a "jerk-like" movement.

For example, with reference to figures 2, 3, 4 and 6, the locking members "B" can comprise a plurality of protuberances 62 and the support element 5 is provided with a tab "L" (shown in figures 2, 3, 4 and 5) intended to rest on the protuberances 62 to define the predefined positions of the support element 5, and to slide along the protuberances 62 during a sliding of the support element 5 along the gripping element 4 to define a passage between a first and a second predefined position.

In other words, thanks to the protuberances 62, the support element 5 is movable "in jerks" between predefined positions.

Preferably, as shown in the appended figures, the protuberances 62 are obtained in the form of teeth arranged inside the channel 61, or in the form of teeth obtained on the rails 6a.

Advantageously, it is possible to size the positioning of the protuberances 62 to allow an operator to make minimum adjustments during the examination of a cavity of a patient.

In more detail, the support element 5 is sliding along the gripping element 4 between an insertion position, an acquisition position and an observation position.

Therefore, the locking members "B" are configured to define the insertion, acquisition and observation positions in which to retain the support element 5 along the gripping element 4. In the insertion position, shown in figure 2, the support element 5 is arranged in a distal position with respect to the dilator body 2, and in particular with respect to the proximal opening "PO".

Advantageously, the insertion position allows an easy and facilitated insertion procedure of the dilator body 2 inside the cavity of a patient. In particular, the operator can insert the dilator body 2 (into the cavity of a patient) and extract the introducer body 3 upon completing insertion of the dilator body 2, without the encumbrance of the support element 5.

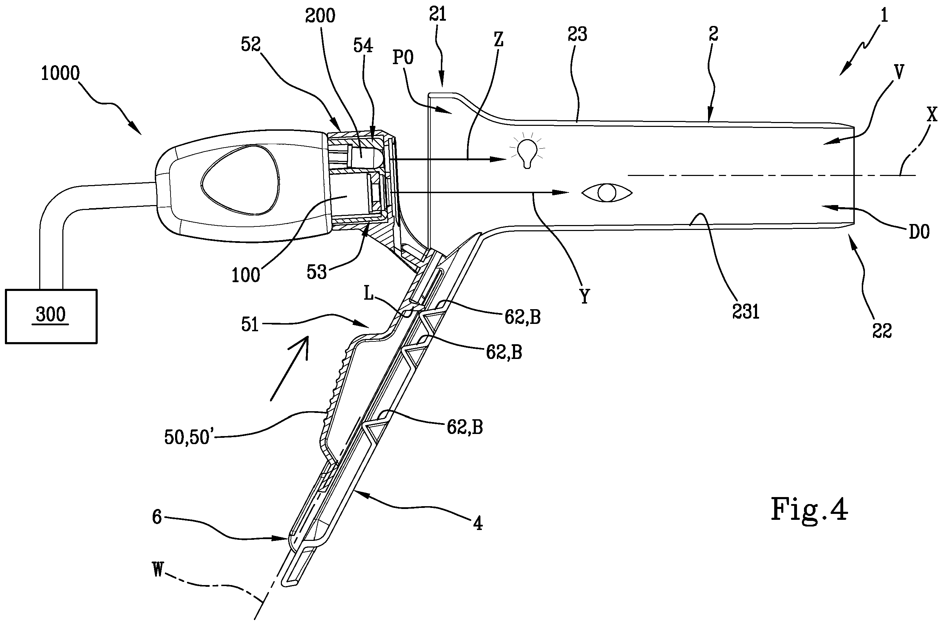

In the acquisition position, shown in figure 4, the support element 5 is arranged in a proximal position with respect to the dilator body 2, and in particular with respect to the proximal opening "PO".

Therefore, the operator, once the dilator body 2 has been inserted into the cavity of a patient and once the introducer body 3 has been disengaged from the inner volume "V", can move the support 5 along the gripping element 4 up to the acquisition position, connect the inspection group 1000, i.e. the acquisition 100 and lighting 200 devices, to the support element 5 and acquire images of the cavity of the patient in a facilitated manner.

Therefore, the device 1 advantageously allows a facilitated acquisition of images of the cavity of a patient.

Advantageously, the times linked to the examination of the cavity of the patient are shorter.

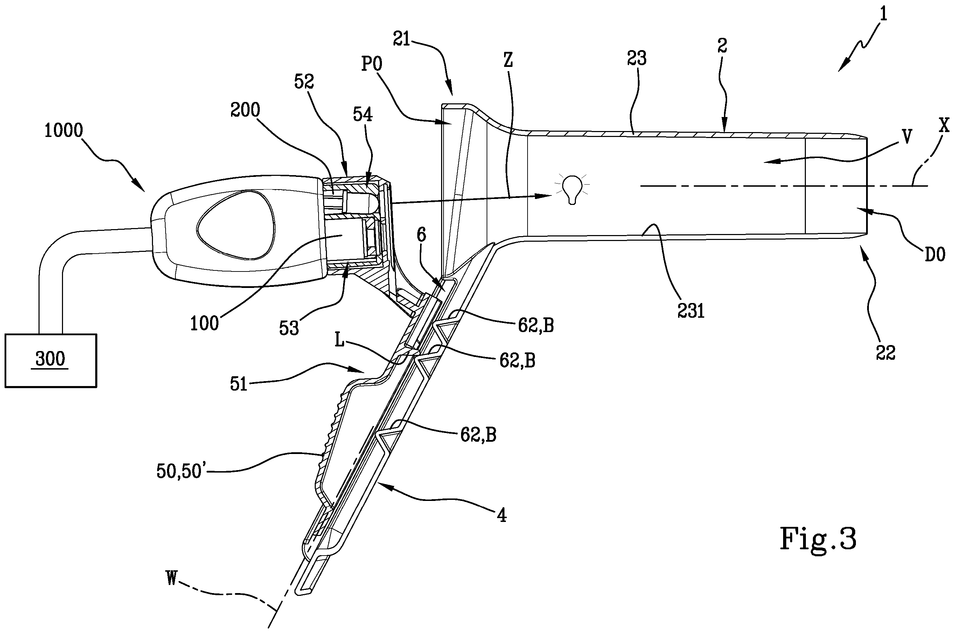

In the observation position, shown in figure 3, the support element 5 is arranged between the insertion position and the acquisition position.

Therefore, in order to observe the cavity of a patient with the naked eye, i.e. without the assistance of the acquisition device 100, the operator can move the support element 5 from the insertion position to the observation position, or from the acquisition positive to the observation position, in an easy and facilitated manner.

The acquisition device 100, and also the lighting device 200, are connectible in a removable manner to the support element 5, so an operator can remove the acquisition device 100 when the support element 5 is in the observation position.

Advantageously, the device 1 allows, in addition to a facilitated acquisition of images of the cavity of a patient, also direction observation (with the naked eye) of it.

Thanks to the possibility of moving the support element 5 between the acquisition and observation positions, the operator can advantageously alternate a visual inspection (with the naked eye) of the cavity of the patient, by positioning the support element 5 in the observation position, and an on-screen inspection (by means of the acquisition device 100), by positioning the support element 5 in the acquisition position.

In other words, the device 1 allows an in-depth inspection of the cavity of a patient.

Also in other words, the device 1 allows the cavity of a patient to be inspected using different methods (with naked eye and on-screen).

In order to allow, in both the lighting position and also in the acquisition position, a correct lighting of the mucous membrane of the patient, operated by means of the lighting device 200 connected to the support element 5, the inner surface 231 is preferably embossed so as to deviate or diffuse the light beam produced by the lighting device 200.

In more detail, in order to allow inspection using different methods, the acquisition seat 53 of the support element 5 extends along a respective acquisition axis "Y" (shown schematically in figure 4 with an arrow) passing through the distal opening "DO" of the dilator body 2 in the acquisition position, whereas its lighting seat 54extends along a respective lighting axis "Z" (shown schematically in figure 3 and in figure 4 with an arrow) passing at least through the proximal opening "PO" of the dilator body 2 both in the acquisition position and in the lighting position. Preferably, the lighting axis "Z" is also passing through the distal opening "DO" of the dilator body 2 in the acquisition and lighting positions.

In particular, the acquisition seat 53 is configured to receive by insertion the acquisition device 100 oriented along a respective acquisition direction, shown in figure 4 in the same manner as the acquisition axis "Y". Therefore, in accordance with the embodiment shown in the appended figures, the acquisition direction is parallel to and coinciding with respect to the acquisition axis "Y".

In accordance with further alternative embodiments not shown in the appended figures, the acquisition direction is parallel to and not coinciding with respect to the acquisition axis "Y", or incident with respect to the acquisition axis "Y".

The acquisition seat 53 is configured to receive by insertion the acquisition device 100 oriented along the respective acquisition direction, so that the acquisition direction is passing through the distal opening "DO" of the dilator body 2 and incident with respect to the longitudinal axis "X" in the acquisition position.

In other words, the acquisition direction is at least partially passing inside the inner volume "V" in the acquisition position, allowing the operator to film the inner volume "V" by means of the acquisition device 100.

The acquisition seat 53 is also configured to receive by insertion the acquisition device 100 oriented along the respective acquisition direction, so that the acquisition direction is neither passing through the distal opening "DO" of the dilator body 2, nor incident with respect to the longitudinal axis "X" in the insertion position.

Thus, the acquisition direction is, in the insertion position, completely extraneous to the inner volume "V", since, in that position, the operator inserts the device 1 inside the cavity of the patient.

Preferably, the acquisition seat 53 is configured to receive by insertion the acquisition device 100 oriented along the respective acquisition direction, so that the acquisition direction is neither passing through the distal opening "DO" of the dilator body 2, nor incident with respect to the longitudinal axis "X" also in the observation position.

Thus, the acquisition direction is preferably, in the observation position, completely extraneous to the inner volume "V", since, in that position, the operator observes the inner volume "with the naked eye" (without the assistance of the acquisition device 100).

With reference to the lighting seat 54, it is configured to receive by insertion the lighting device 200 oriented along a respective lighting direction, shown in figures 3 and 4 in the same manner as the lighting axis "Z".

Therefore, in accordance with the embodiment shown in the appended figures, the lighting direction is parallel to and coinciding with respect to the lighting axis "Z".

In accordance with further alternative embodiments not shown in the appended figures, the lighting direction is parallel to and not coinciding with respect to the lighting axis "Z", or incident with respect to the lighting axis "Z".

The lighting seat 54 is configured to receive by insertion the lighting device 200 oriented along the respective lighting direction, so that the lighting direction is passing through the distal opening "DO" of the dilator body 2 and incident with respect to the longitudinal axis "X" in the acquisition position and in the lighting position.

In other words, the lighting direction is at least partially passing inside the inner volume "V" in the acquisition and observation position, allowing the operator to light the inner volume "V" by means of the lighting device 200. The lighting seat 54 is also configured to receive by insertion the lighting device 200 oriented along the respective lighting direction, so that the lighting direction is neither passing through the distal opening "DO" of the dilator body 2, nor incident with respect to the longitudinal axis "X" in the insertion position.

Thus, the lighting direction is, in the insertion position, completely extraneous to the inner volume "V", since, in that position, the operator inserts the device 1 into the cavity of the patient.

The term "completely extraneous to" is intended to mean that the acquisition/lighting direction is not passing inside the inner volume "V". The present invention also provides an inspection kit of a cavity.

The kit comprises the device 1 and the inspection group 1000, i.e. the acquisition device 100 and the lighting device 200.

Preferably, the acquisition device 100 is in the form of a fibre-optic camera.

Preferably, the lighting device 200 is made in the form of a fibre-optic illuminator.

The kit further comprises a portable battery 300, connected or connectible, for example by means of specific cables, to the acquisition device 100 and/or to the lighting device 200.

Preferably, the portable battery 300 is connected or connectible both to the acquisition device 100 and to the lighting device 200.

For example, the battery 300 can be the battery of a portable device such as a computer or a tablet, making the kit versatile to use. Advantageously, the portable battery 300 allows a lower cost of the kit and easy use of it.

Furthermore, since the portable battery 300 is external and separate with respect to the inner volume "V" of the dilator body 2, it does not have to be discarded after every use, so the kit is sustainable.

In accordance with an aspect of the present invention, thanks to the support element 5, the centre of gravity of the kit in a use configuration, when the acquisition device 100 and the lighting device 200 are connected to the support element 5, is positioned proximal to the support element 5. Advantageously, the kit therefore allows an operator to support and to move the kit in a facilitated manner.

Advantageously, the present invention is capable of overcoming the drawbacks which have emerged from the prior art.

Advantageously, the device 1 allows a facilitated inspection of the cavity of a patient, thanks to the support element 5, and also thanks to the guide element 6, allowing simultaneously the performance of an examination on a patient that is not too uncomfortable and of limited duration.

In addition, advantageously, the device 1 allows facilitated adjustment of the position of the acquisition device 100 and the lighting device 200, thanks to the locking members "B" comprising, for example, the protuberances 62.

Furthermore, the device 1 and the kit are sustainable.

## Claims

1. An inspection device (1) of a cavity, comprising:
- a dilator body (2), having a prevalent extension along a longitudinal axis (X) between a proximal end (21) and a distal end (22), wherein the proximal end (21) and the distal end (22) are connected by a side wall (23) and define, together with said side wall (23), an inner volume (V), wherein the proximal end (21) identifies a proximal opening (PO), and wherein the distal end (22) identifies a distal opening (DO) for access, from the inner volume (V), to the tissues delimiting the cavity;
- an introducer body (3), applicable in use in a removable manner to said dilator body (2), in particular the introducer body (3) being slidably insertable into, and disengageable from, the inner volume (V) through the proximal opening (PO);
- a gripping element (4) with an elongated shape, applied or applicable to the proximal end (PO) of the dilator body (2), wherein the gripping element (4) is arranged transversely with respect to the longitudinal axis (X) and wherein the gripping element (4) is graspable by an operator,
said device (1) being **characterised in that** it comprises a support element (5) configured to define a reversible connection with an inspection group (1000),
wherein the support element (5) is arranged externally with respect to the inner volume (V) defined by the dilator body (2), so as to support the inspection group (1000) externally with respect to said inner volume (V), and
wherein the support element (5) is connected to the gripping element (4) and is movable along said gripping element (4) towards and/or away from the proximal end (PO),
the inspection group (1000) comprising at least one acquisition device (100) and at least one lighting device (200).

2. The device (1) according to claim 1, comprising a guide element (6), arranged at the gripping element (4), configured to guide a movement of the support element (5) along a predefined path, extending along the gripping element (4).

3. The device (1) according to claim 2, wherein the guide element (6) is defined on the gripping element (4), the guide element (6) preferably being in the form of at least one rail (6a) defined on the gripping element (5).

4. The device (1) according to claim 2, wherein the guide element (6) is applied in a removable manner to the gripping element (4), the guide element (6) preferably being in the form of at least one rail (6a) applied or applicable in a removable manner to the gripping element (4).

5. The device (1) according to any one of claims 2-4, wherein the guide element (6) defines a channel (61) delimiting the predefined path, and wherein the support element (5) comprises:
- an interconnection portion (51), inserted into, and sliding along, said channel (61), and
- a connection portion (52) extending transversely with respect to said channel (61) or with respect to said interconnection portion (51) and configured to support the inspection group (1000).

6. The device (1) according to any one of claims 2-5, wherein the guide element (6) comprises locking members (B) configured to define a respective plurality of predefined positions in which to retain said support element (5) along said gripping element (4), said locking members (B) preferably comprising a plurality of protuberances (62).

7. The device (1) according to any one of the preceding claims, wherein the support element (5) is sliding along the gripping element (4) between:
- an insertion position, in which the support element (5) is arranged in a distal position with respect to the proximal opening (PO) of the dilator body (2);
- an acquisition position, in which the support element (5) is arranged in a proximal position with respect to the proximal opening (PO) of the dilator body (2);
- an observation position, in which the support element (5) is arranged between the insertion position and the acquisition position.

8. The device (1) according to any one of the preceding claims, wherein the support element (5) comprises:
- an acquisition seat (53), configured in use to receive the acquisition device (100) by insertion,
- a lighting seat (54), configured in use to receive the lighting device (200) by insertion,

9. The device (1) according to claims 5 and 8, wherein said acquisition seat (53) and said lighting seat (54) are defined on said connection portion (52) and pass-through with respect to the connection portion (52).

10. The device (1) according to claim 8 or 9, when dependent on claim 7, wherein:
- the acquisition seat (53) extends along a respective acquisition axis (Y) passing through the distal opening (DO) of the dilator body (2) in the acquisition position,
- the lighting seat (54) extends along a respective lighting axis (Z) passing through at least the proximal opening (PO) of the dilator body (2) in both the acquisition position and in the lighting position, the lighting axis (Z) preferably also passing through the distal opening (DO) of the dilator body (2).

11. The device (1) according to claim 10, wherein the acquisition seat (53) defines in use an acquisition direction parallel to or coincident with the acquisition axis (Y) and is configured in use to receive by insertion the acquisition device (100) of the inspection group (1000) oriented along the respective acquisition direction; said acquisition direction or said acquisition axis (Y) being:
- passing through the distal opening (DO) of the dilator body (2) and incident with respect to the longitudinal axis (X) in the acquisition position, and
- not passing through the distal opening (DO) of the dilator body (2) and not incident with respect to the longitudinal axis (X) in the insertion position.

12. The device (1) according to claim 10 or 11, wherein the lighting seat (54) defines in use a lighting direction parallel to or coincident with the lighting axis (Y) and is configured in use to receive by insertion the lighting device (200) of the inspection group (1000) oriented along the respective lighting direction;
said lighting direction or said lighting axis (Z) being:
- passing through the distal opening (DO) of the dilator body (2) and incident with respect to the longitudinal axis (X) in the acquisition position and in the lighting position, and
- not passing through the distal opening (DO) of the dilator body (2) and not incident with respect to the longitudinal axis (X) in the insertion position.

13. The device (1) according to any one of the preceding claims, wherein the support element (5) comprises (5) comprises an adaptor (55), configured to define a reversible connection with the inspection group (1000), the adaptor (55) being removable from said support element (5).

14. An inspection kit of a cavity, comprising:
a device (1) according to any one of the preceding claims;
- an acquisition device (100) and a lighting device (200) reversibly connected to the support element (5) of the device (1);
- a portable battery (300) connected or connectible to the acquisition device (100) and to the lighting device (200).
